# EUROPEAN PATENT SPECIFICATION

(11) **EP 0 605 427 B1**
(45) Date of publication and mention of the grant of the patent: **20.05.1998**
(21) Application number: 92913394.0
(22) Date of filing: 05.06.1992
(51) Int. Cl.: A61M 25/00

(54) **NON-CONTAMINATING PROBE**
NICHTINFIZIERENDE SONDE
SONDE NON CONTAMINANTE

(30) Priority: 07.06.1991 IT PS910027 U
(43) Date of publication of application: 13.07.1994
(73) Proprietor: RTC INC., St. Paul, MN 55105 (US)
(72) Inventor: FELIZIANI, Roberto, I-61037 Mondolfo (IT); JAKER, Marc, L., New Brighton, MN 55112 (US)
(74) Representative: Patentanwälte Wenzel & Kalkoff
(86) International application number: US9204727
(87) International publication number: WO9221399

(56) References cited:
- EP-A- 0 247 559
- DE-A- 1 958 561
- US-A- 3 084 693
- US-A- 3 332 424
- US-A- 3 421 509
- US-A- 3 866 601

## Description

### Field of The Invention

The present invention relates, generally, to sterilized medical probes and, more particularly, to an anti-infectious Prophylactic probe useful as a urinary catheter. Accordingly, the subject matter of the present invention refers to a catheter for insertion into an anatomical canal.

### Background of the Invention

Catheters or probes are used in a number of medical and related applications. For example, a catheter comprising an elongated, flexible hollow tube may be inserted into a patient's urethral canal to permit withdrawal of fluids from the bladder. Such catheters are typically referred to as urinary drainage catheters, or simply urinary catheters, and are usually inserted into the bladder by way of the urethra.

It has been found that typical urinary catheters, even if sterile when inserted into a patient, tend to contribute to bladder infections. Treatment of such infections often involves the use of heavy medication and may lengthen the catheterized patient's hospital stay. It is believed that the insertion of the catheter through the urethral canal into the bladder dislodges or otherwise attracts infectious material, for example pathogens and bacteria which populate the lower urinary tract, due to the frictional forces the catheter exerts on the urethral walls or urethral mucosa resident on the walls. These bacteria are typically present on the male glans or the distal portion of the male's urethra, or on the female's urethral meatus. Once dislodged, the infectious material is carried, typically by the leading edge of the catheter, to the internal organ (*e.g*. bladder), causing infection of the internal organ.

Even if no infectious or pathogenous material is dislodged, the frictional forces exerted by the catheter on the urethral walls by conventional catheters tend to irritate the urethral mucosa. While the irritation caused by this mechanical trauma exerted during penetration of the catheter is generally temporary, the discomfort experienced by the patient is nonetheless substantial.

These disadvantages are also encountered in other applications in which a probe or catheter is inserted into a body cavity to either drain or inject internal fluids or for other diagnostic procedures. For example, probes used in cystoscopy also may cause attraction of infectious material or irritation to the canal or orifice into which the catheter is inserted.

There thus exists a long felt need to ameliorate the disadvantages occasioned by use of known medical probes. There is a particular need for a urinary catheter which does not cause infectious material to be carried or otherwise passed to the bladder.

According to German patent application 1,958,561 A which corresponds substantially to US-patent specification 3,583,391 a urinary catheter is known which includes an elongated, flexible sac for positioning same in the urethral canal before and during insertion of a catheter tube. The purpose of the sac is to prevent the walls of the urethral canal from contacting the catheter tube when the catheter tube is pushed forward in the urethral canal so as to avoid that infectious matter enters the bladder as the catheter tube travels to the bladder. The elongated sac fits in a bore of a flexible insertion tube which functions as catheter tube as well. One end of the sac extends from a front end of the flexible insertion tube and is secured to a rigid tubular guide sleeve encircling a considerable portion of the flexible insertion tube. The rigid tubular sleeve forms a close fitting guide arrangement which provides the required columnar rigidity to the flexible insertion tube, but does not itself enter the urethral. Accordingly, this known system functions like a telescopic guide or like a piston in a cylinder so as to avoid bending and buckling of the flexible insertion tube. As the flexible insertion tube is pushed forward, the insertion tube extends out of the guide sleeve into the urethra and the sac rolls out over a front end of the insertion tube into contact with the urethra. Application of lubricants is used for aiding in rolling the sac over the open end the flexible insertion tube and for this purpose the inner and outer surface of the insertion tube is coated with silicone oil.

Since the rigid tubular guide sleeve covers a long portion of the flexible insertion tube there is no access to the insertion tube for pushing it forward by hand in the urethral canal. For providing an access to the insertion tube slots are provided which extend on opposite sides of the rigid guide sleeve along a considerable portion of said sleeve. A pair of diametrically opposed handles being fastened to the flexible insertion tube extend through the slots. The handles can be grasped for pushing the flexible insertion tube forward.

Before use all parts, namely the flexible sac and the flexible insertion tube must be effectively sterilized inside and outside. Also the rigid tubular guide sleeve must be sterilized inside.

Due to the complex structure of the known catheter a complete and effective sterilization requires extraordinary sterilization methods which allow even maintenance of the sterilization effect also during mounting of the catheter and until use of the catheter. Mainly because of the relatively high resistance of the sac to slide over the leading edge of the flexible insertion tube this known catheter has not been applied in practice.

### Summary of the Invention

The present invention provides an anti-infectious, non-contaminating catheter which addresses the aforementioned drawback of presently known catheters and which comprises the features of claim 1.

A preferred exemplary embodiment of the present invention provides, *inter alia*, a generally hollow tube having an axial lumen extending along the length thereof, and an elongated, tubular elastic membrane disposed within the lumen, wherein the leading end of the membrane is circumferentially folded back over the leading edge of the tube such that as the catheter is inserted into a patient, the membrane is drawn through the tube and out the leading edge of the tube. Once the catheter is fully inserted, the membrane circumscribes the tube; thus, contact between the tube and the patient is substantially, if not entirely, prevented during insertion of the catheter.

More particularly, the non-contaminating catheter of the present invention includes a protective membrane (sheath) which is laid out as the catheter is urged along an anatomical canal and thus remains interposed between the exterior wall of the catheter and the walls of the canal (*e.g*., the urethral canal). In accordance with one aspect of the invention, a catheter tube suitably cooperates with a membrane such that upon insertion of the catheter into an orifice, such as the urethral canal, sliding frictional contact between the catheter and the urethral mucosa is inhibited and the passive transportation of pathogens in the lower urinary tract concomitantly prevented.

The apparatus of the present invention thus facilitates insertion of a catheter into a body cavity in a manner which effectively reduces the irritation caused by the frictional forces exerted by typical catheters and inhibits the dragging of pathogens to internal organs during the catheterization procedure. A method of using the catheter of the present invention is also disclosed.

In subclaims 2-15 improvements of the catheter according to claim 1 are specified.

### Brief Description of the Drawing

Preferred exemplary embodiments of the present invention will hereinafter be described in conjunction with the appended drawing figures, wherein like numerals denote like elements and:
Figure 1 is a side elevational view of a preferred embodiment of a catheter in accordance with the present invention;
Figure 2 is an elevational view of the catheter of Figure 1 during an initial insertion into a patient;
Figure 3 is an elevational view of the catheter of Figures 1 and 2 upon further insertion into a patient;
Figure 4 is a perspective view of an alternative embodiment of a catheter in accordance with the present invention;
Figure 5A is a sectioned perspective view showing one type of catheter tubing useful in accordance with the present invention;
Figure 5B is a sectioned perspective view showing an alternate type of catheter tubing useful in accordance with the present inventions;
Figure 5C is a sectioned perspective view showing catheter tubing provided with ribs in accordance with the present invention;
Figure 6 is a cross-sectional view of a guide ring useful in the catheter in accordance with the present invention.

### Detailed Description of Preferred Exemplary Embodiments

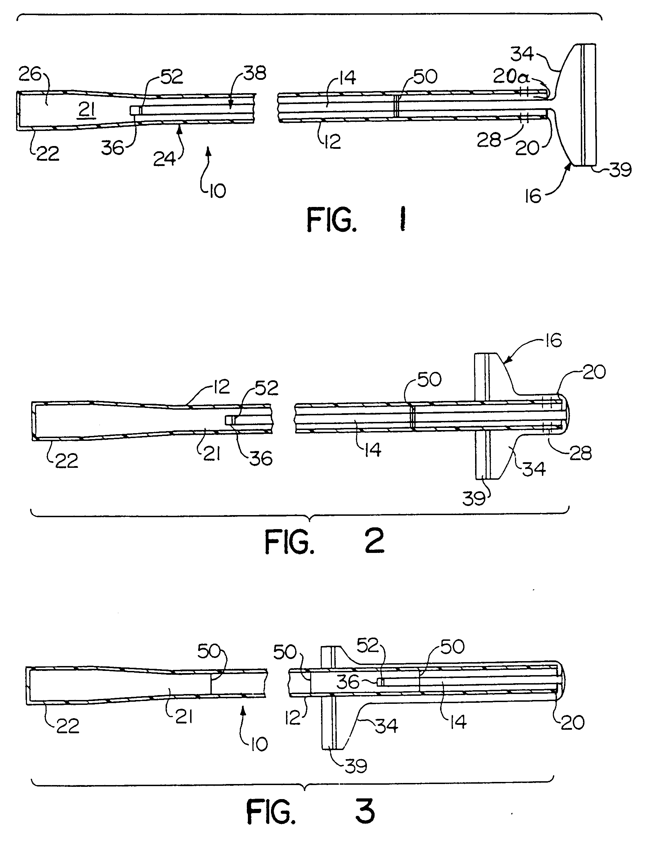

Referring now to Figure 1, an exemplary non-contaminating catheter 10 in accordance with the present invention preferably includes a catheter tube 12, a membrane (sheath) 14, and a membrane guide 16. Membrane 14 suitably comprises a first end 34, a second end 36, and a flexible body 38 spanning therebetween.

Catheter tube 12 may be of a conventional design and typically includes a leading edge 20, a trailing edge 22, with a generally tubular body 24 and an axial bore (lumen) 21 extending therebetween. As best seen in Figures 1-3, tube 12 may have a diameter which varies from leading edge 20 to trailing edge 22. The diameter of tube 12 in the area of leading edge 20 is advantageously in the range of about 5 to about 6mm. Preferably, the diameter of tube 12 varies at about 0.33mm increments from leading edge 20 to trailing edge 22. When catheter 10 is used as a urinary catheter, the outer diameter of tube 12 is advantageously in the range of about 5,08 to 7,6 mm (0.20 to 0.30 inches), typically about 6,35 mm (0.25 inch). The diameter of lumen 21 of tube 12 is thus preferably in the range of about 2,54 to about 5,08 mm (0.10 to about 0.20 inch).

Tube 12 preferably has a length characteristic of conventional catheters, tubes or probes. For example, when catheter 10 is used as a urinary catheter for males, it will generally have a length of from about 30 to about 50 cm, typically about 40 cm; when utilized as a urinary catheter for females, tube 12 generally will have a length of from about 15 to about 30 cm, typically about 20 cm. Preferably, tip 20a of tube 12 is configured to have a very smooth configuration, most preferably having a radius in the range of 1,72 mm (0.050 inches) for a tube having a diameter of about 6,35 mm (0.25 inches).

Tube 12 preferably includes at least one aperture 28 provided in body 24 proximate leading edge 20 of tube 12. As will be discussed more fully in the following description, aperture(s) 28 permits fluid to enter lumen 21 to facilitate drainage of bodily fluids from the patient. Apertures 28 may be provided in body 24 of tube 12 in any conventional fashion such as by drilling or the like. Tube 12 may also be provided with one or more enlargements or balloons, or a double balloon (all not shown) to enable catheter 10 to be maintained permanently in place inside the bladder. As is known in the art, such balloons or inflatable bags may be inflated when the catheter has been advanced to its desired position to ensure retention of the catheter in such position and prevent its accidental withdrawal.

Trailing edge 22 of tube 12 preferably includes a coupler 26 securely affixed thereto. Coupler 26 is suitably configured to facilitate adaption of tube 12 to the opening of a conventional urine collection bag or the like. As shown in Figure 1, coupler 26 suitably comprises an enlarged portion of tube 12 proximate trailing edge 22. Alternatively, a coupler element (not shown) may be suitably attached to trailing edge 22 of tube 12 by any conventional means.

The particular material comprising tube 12 desirably exhibits sufficient strength to ensure that tube 12 does not collapse during insertion, as well as sufficient elasticity to permit maneuvering of catheter 10 during the catheterization process. Thus, tube 12 is preferably made from a clear, flexible polymeric material, for example an FDA certifiable material suitable for *in vivo* use. In accordance with a particularly preferred embodiment, tube 12 comprises a polymeric material having a Shore A hardness in the range of about 70 to 160, and preferably about 90. Suitable polymeric materials include thermoplastic polymers, for example polyurethanes, polyvinyl chlorides, and the like (*e.g*., medical grade polyvinyl chloride). Such thermoplastic polymers may be natural or synthetic and may be modified by the addition of lubricants such as glycerine, glycol and the like. Other conventional additives now known or hereafter devised by those skilled in the art may also suitably be included. Tube 12 may also comprise fluorocarbon polymers such as fluorinated ethylene-propylene resins or tetrafluoroethylene commonly referred to by the trademark Teflon owned by E.I. duPont de Nemours & Company. A particularly preferred material for tube 12 is a Teflon tubing having a Shore A durometer in the range of about 90-95, which is available from Medical Profiles, Lavonia, Michigan.

Leading edge 20 of tube 12 may optionally be comprised of a more rigid material than the remainder of tube 12. As explained in greater detail hereinafter, as catheter 10 is inserted into the patient, membrane 14 is caused to fold over and escape the interior of tube 12 about the tip 20a of tube 12. Thus, in order to avoid dilation of tip 20a, it may be comprised of a nonelastic material such as Teflon, nylon, acetal, or the like. Providing tip 20a with enhanced rigidity also tends to further facilitate the sliding between membrane 14 and tube 12.

Membrane 14 preferably comprises a thin elastic polymeric material advantageously having sufficient lubricity to smoothly slide out of and over tip 20a of leading edge 20 and subsequently slide back along the exterior of tube 12. Membrane 14 may suitably comprise any conventional thermoplastic polymer such a polyethylene (low or high density). Those skilled in the art will appreciate that conventional additives such as ethylene-vinyl acetate copolymer, vinyl additives, fluorinated ethylene-propylene resins and the like may be added to increase strength or other properties of the membrane. Membrane 14 may also suitably comprise synthetic resins, such as polyesters and synthetic resin alloys such as polytetrafluoroethylene. Natural or synthetic rubbers such as polyisoprene, polychloroprene, ethylenepropylene-diene may also be employed to form membrane 14. A particularly preferred membrane material is a natural latex having a wall thickness in the range of about 0,20 to about 0,254 mm (0.008 to about 0.010 inches) such as is available from Menick Corporation, Minneapolis, Minnesota. It should be understood, however, that any suitable membrane material having sufficient strength, integrity, elasticity and lubricity may be utilized in accordance with the present invention to form membrane 14, and the specific forms disclosed herein are provided for illustration only and not in any limiting sense.

The material comprising membrane 14 also may be provided with a lubricant to increase the ease with which membrane 14 is folded over tube 12 during use. For example, 0 to about 30% of a conventional lubricant may be loaded during manufacture of membrane 14. Suitable lubricants include corn starch, starch, talcum, calcium carbonate, glycerin, silicone, limestone, and the like. For example, membrane 14 may comprise polyethylene with about 15% corn starch. Corn starch is believed to be a suitable additive in that it is generally hypo-allergenic and suitable for *in vivo* use.

Lubricant may also be optionally applied to the external surfaces of membrane 14. This enables easy insertion of membrane 14 into tube 12 prior to use, smooth extraction of membrane 14 from tube 12 upon insertion of catheter 10 *in vivo*, and smooth insertion of catheter 10 into the cavity, *e.g*. the urethra.

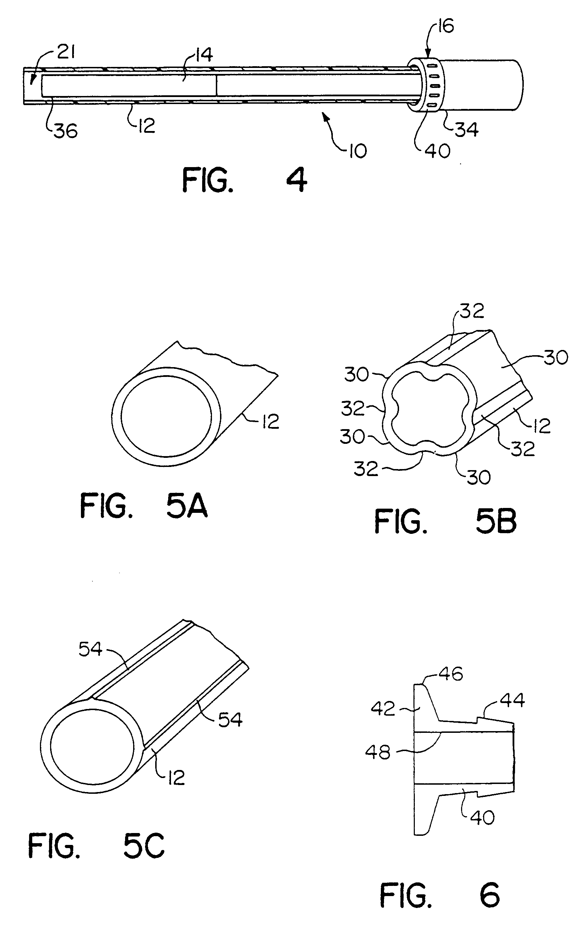

With momentary reference to Figure 5C, membrane 14 may optionally be provided with one or more, preferably two or more, ribs 54 extending about the exterior surface of body 38 as an alternative to the use of lubricants. Ribs 54 facilitate extraction of membrane 14 from tube 12 as catheter 10 is inserted into a cavity. More particularly, ribs 54 tend to reduce the frictional forces exerted between membrane 14 and tube 12 during their relative sliding engagement by reducing the area of contact between the two elements. Ribs 54 also tend to increase the strength of membrane 14 to prevent rips or tears during use of catheter 10.

While Figure 5C illustrates ribs 54 in conjunction with tube 12 having a circular cross section, ribs 54 may also be advantageously employed with tubes manifesting various geometries, for example the cloverleaf configuration of Figure 5B. Ribs 54 are suitably formed by conventional techniques during the manufacture of membrane 14 or by affixation subsequent to manufacture. In the event ribs 54 are provided post-manufacture, suitable filaments such as nylon/polyester filaments may be applied through the use of conventional adhesives such as medical grade bonding agents. The size of filaments forming ribs 54 may vary depending on the particular membrane film utilized as well as the strength, flexibility and lubricity of the filament. Filaments in the range of about 0,254 mm (0.010 inches) in diameter typically yield performance.

Those skilled in the art will appreciate, however, that the various cooperating components comprising catheter 10 may be formed of any suitable material, and that the preceding examples are merely illustrative.

Membrane 14 preferably comprises a substantially uniform cylindrical tube. Second end 36 of membrane 14 (which is initially within tube 12) is open.

Membrane 14 typically exhibits a length less than that of tube 12. For example, if catheter 10 is used as a urinary catheter, membrane 14 is desirably in the range of about 10 to about 40 cm, and optimally about 20 cm in length for use with males; membrane 14 is desirably in the range of about 10 to about 25 cm, and optimally about 15 cm for use with females.

A plurality of calibrations 50, *i.e*. one or more lines, are suitably imprinted on the exterior surface of tube 12. Similarly, a plurality of calibrations 52, *i.e*. one or more lines, are imprinted on the interior surface of membrane 14. The cooperation of calibrations 50 and 52 enable the user to determine the position of second end 36 of membrane 14 as catheter 10 is inserted into a body cavity.

Tube 12 may assume any conventional configuration. In the embodiment shown in Figure 5A, tube 12 is generally circular in cross section. In a more preferred embodiment, however, tube 12 suitably comprises a shaped tube, for example the generally cloverleaf configuration shown in Figure 5B. In accordance with the embodiment of Figure 5B, tube 12 includes alternating lands 30 and valleys 32. Lands 30 and valleys 32 cooperate to enhance the unfolding of membrane 14 from tube 12.

Referring now to Figures 1-4, membrane 14 is advantageously disposed, prior to insertion of the catheter into the patient, such that substantially all of membrane 14 is contained within lumen 21, with the exception of first end 34 which engages membrane guide 16. Membrane guide 16 is securely affixed to first end 34 of membrane 14, and is initially disposed about the exterior of leading edge 20 of tube 12. Second end 36 of membrane 14 is thus disposed a considerable distance within tube 12. As will be discussed more fully in connection with Figures 2 and 3, during the catheterization procedure, second end 36 of membrane 14 travels through lumen 21 towards leading edge 20 of tube 12. In accordance with one aspect of the invention, second end 36 is desirably drawn completely through leading edge 20, such that membrane 14 is turned completely inside-out and circumscribes tube 12.

During assembly of catheter 10, membrane 14 is inserted into tube 12 in any suitable manner. For example, membrane 14 may be threaded through lumen 21 of tube 12. Alternatively, membrane 14 may be pneumatically or hydraulically inflated to facilitate guiding the membrane through tube 12. Second end 36 of membrane 14 is thus disposed within tube 12 and first end 34, together with membrane guide 16, is disposed exteriorly of tube 12. Prior to use, membrane guide 16 and first end 34 are pulled back over leading edge 20 of tube 12 as shown in Figures 2 and 4.

With continued reference to Figures 1-4, membrane guide 16 preferably comprises an enlarged portion 39 which facilitates adaptation of membrane guide 16 to the external glans of the patient during insertion of catheter 10. When catheter 10 is used as a urinary catheter for males, for example, enlarged portion 39 is suitably configured to facilitate holding membrane 14 firmly in place over the glans of the penis during insertion of the catheter.

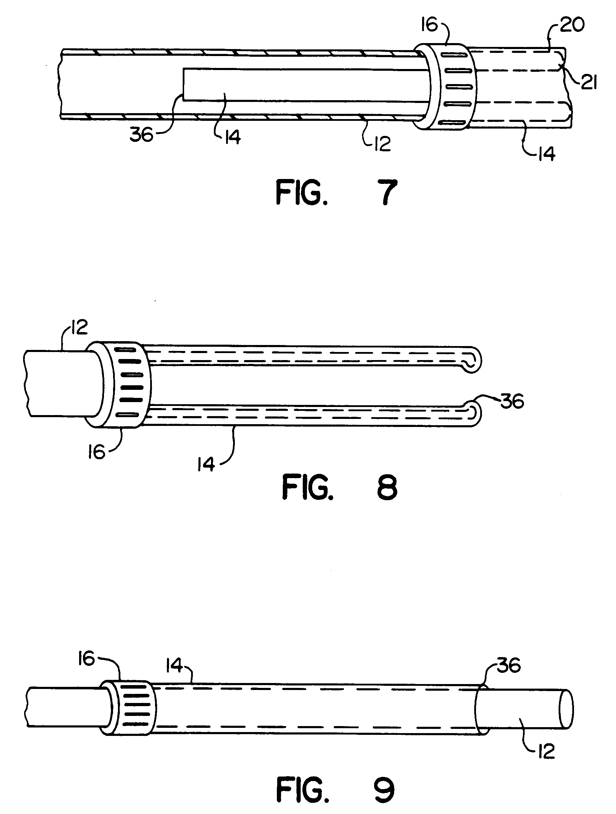

If, however, catheter 10 is configured for use with females, or if it is desired to manually grasp end 34 of membrane 14 while the catheter is in place, membrane guide 16 may be suitably configured to permit the user to grasp membrane guide 16. With particular reference to Figures 4 and 6, membrane guide 16 may alternately be configured in the shape of a ring 40 preferably having a knurled exterior surface. With reference to Figure 6, ring 40 preferably includes an inner bearing surface 48, an outwardly extending flange 42, and a connecting ridge 44. Preferably, flange 42 carries a plurality of knurls about the outer most edge 46. Connecting ridge 44 is suitably configured such that membrane 14 can be securely affixed thereto.

The outer diameter of ridge 44 (or the connecting portion of enlargement 39) is preferably sufficiently larger than the inner diameter of membrane 14 such that a "force-fit" engagement between membrane 14 and membrane guide 16 is maintained. Optional adhesives and mechanical fasteners, such as a snap ring, may be utilized to ensure secure attachment of membrane 14 to membrane guide 16. Alternatively, membrane 14 may be rubber adhesive bonded to membrane guide 16 in accordance with conventional techniques.

Membrane guide 16 may comprise any suitable material such as metal, plastic or the like. Membrane guide 16 suitably comprises a material having a relatively low frictional coefficient, such as polyethylene or polytetrafluoroethylene based materials. Irrespective of the particular material used for membrane guide 16, the inner bearing surface 48 thereof optimally exhibits sufficient lubricity to easily pass along the outside of tube 12 during use.

As will be appreciated, the respective sizes of membrane guide 16 and membrane 14 are chosen to facilitate their convenient movement over the exterior surface of tube 12. For example, if the diameter of tube 12 at leading edge 20 is in the range of about 5mm, then the diameter of membrane 14 and the inner diameter of membrane guide 16 in accordance with one aspect of the invention should be at least about 6mm.

Returning now to Figures 1-3, the operation of a first exemplary embodiment of the present invention will now be described. As best seen in Figure 1, catheter 10 is initially assembled such that second end 36 of membrane 14 is disposed within tube 12. First end 34 of membrane 14 is affixed to membrane guide 16 and is positioned proximate leading edge 20 of tube 12 . Thereafter, membrane guide 16 is guided back over leading edge 20 to initiate the folding of membrane 14 over tube 12, as shown in Figure 2. That is, the portion of membrane 14 external to tube 12 is turned inside-out such that as it is pulled back (to the left in Figures 1-3) along the length of tube 12, the "folded over" portion of membrane 14 circumscribes tube 12. Catheter 10 is now ready for insertion into a patient.

Insertion of catheter 10 may be accomplished through the use of known catheterization techniques. For example, when using catheter 10 in the catheterization of a male's bladder, the patient (or the attending medical personnel) holds guide 16 firmly in place over the male's penis, with one hand. The other hand is used to introduce the catheter 10 into the urethral canal by pushing it inside the urethra. In particular, tube 12 is grasped and manually urged into the urethral canal, thereby causing enlarged portion 39 to engage the glans and causing membrane 14 to begin to fold back over tube 12 in an inside-out fashion. Further pushing of tube 12 into the urethral canal results in continued extraction of membrane 14 from tube 12 (see Figure 3). As membrane 14 is drawn out of tube 12, it is interposed between tube 12 and the walls of the urethral canal, thereby preventing tube 12 from contacting the urethral mucosa and/or the walls of the urethral canal. Consequently, tube 12 does not urge infectious matter up into the bladder as tube 12 travels to the bladder. This feature represents a significant advancement over prior art catheters.

The user can monitor the degree of insertion of catheter 10, as well as the location of membrane 14, by visual inspection of calibrations 50 of tube 12 in conjunction with calibrations 52 of membrane 14. Once tube 12 has reached the bladder, membrane 14 may, if desired, be extracted by grasping membrane guide 16 and pulling membrane 14 rearwardly until second end 36 of membrane 14 is completely removed from the patent.

It should thus be appreciated that catheter 10 in accordance with the present invention enables a tube or probe to be inserted into an orifice, such as the urethral canal, without transporting pathogens or bacteria normally inhabitant at or near the glans or in the proximity of the lower urethral canal to the bladder, through the use of non-contaminating membrane 14. Those skilled in the art will also appreciate that in addition to its usefulness in catheterization procedures, catheter 10 of the present invention can be utilized as a probe for other known or hereafter devised medical techniques. For example, the probe of the present invention can be used for the drainage of internal fluids. Further, catheter 10 may be used to perform bladder lavages in continuum by injecting large amounts of fluids (*e.g*., water) into the bladder through the use of catheter 10. For example, once catheter 10 is completely inserted and membrane 14 inverted such that end 36 is proximate tip 20a, fluid may be injected into the area between membrane 14 and the exterior surface of tube 12, thus causing membrane 14 to slightly expand. The fluid entering and cleansing the bladder can then be extracted through lumen 21 of tube 12.

It will be understood that the above description is of preferred exemplary embodiments of the present invention, and that the invention is not limited to the specific form shown and described herein. For example, some or all of the components may be plasma surface treated such as with Teflon to enhance hydrofilasticity. Further, various alternative configurations of catheter 10, particularly tube 12, may be readily incorporated by those skilled in the art. These and other modifications may be made in the design and arrangement of the elements within the scope of the invention, as expressed in the appended claims.

## Claims

1. A catheter for insertion into an anatomical canal, the catheter comprising:
- a generally hollow catheter tube (12) having respective oppositely disposed leading and trailing edges (20, 22) , an axial lumen (21) extending through the length thereof,
- the catheter tube (12) having a tip (20a) which includes the leading edge (20) of the catheter tube (12) and which is configured to have a smooth configuration,
- the catheter tube (12) being made of a flexible polymeric material having sufficient strength such that catheter tube (12) does not collapse during insertion as well as sufficient elasticity to permit manoeuvering of the catheter (10) during the insertion into the anatomical canal,
- an elongated tubular and sufficiently elastic membrane (14) disposed within the lumen (21) having first and second open ends (34, 36) and a body (38) spanning therebetween;
- said membrane (14) being made of a thin polymeric material having sufficient lubricity to smoothly slide out of the opening of tip (21a) and over the leading edge (20) of the catheter tube (12) and subsequently slide back relative to the exterior of catheter tube (12),
- a membrane guide (16) being configured in the shape of a ring (40) and suitably configured for axial movement about the exterior of the catheter tube (12), further being made of a plastic material having a relatively low frictional coefficient, first end (34) of membrane (14) being securely affixed to said membrane guide (16),
- the respective sizes of membrane guide (16) and membrane (14) being chosen such that their convenient movement over the exterior surface of catheter tube (12) is facilitated,
- wherein said membrane guide (16) and said membrane first end (34) are disposed about the exterior of said tube leading edge (20), and said membrane second end (36) and a major portion of said membrane body (38) are positioned inside said tube lumen (21), such that as the catheter (10) is manually inserted into the anatomical canal, pushing of catheter tube (12) into said canal will result in continued extraction of membrane (14) from catheter tube (12) so that finally said membrane (14) will be withdrawn from said tube lumen (21) and, be interposed between said tube (12) and said anatomical canal.

2. Catheter according to claim 1, characterized by that the outer diameter of the catheter tube (12), when catheter (10) is used as urinary catheter, is in the range of about 5,08 to 7,6 mm, particularly about 6,35 mm.

3. Catheter according to claim 1 or 2, characterized by that the diameter of lumen (21) of the catheter tube (12) is in the range of about 2,54 mm to about 5,08 mm.

4. Catheter according to one or more of the preceding claims, characterized by that the radius of tip (20) of the catheter tube (12) is in the range of 1,27 mm for a catheter tube (12) having a diameter of about 6,35 mm.

5. Catheter according to one or more of the preceding claims, characterized by that the catheter tube (12) is made of polyvinylchloride (PVC), particularly medical grade polyvinylchloride.

6. Catheter according to one or more of the preceding claims, characterized by that the catheter tube (12) comprises a polymeric material having a Shore A hardness in the range of about 70 to 160, and preferably about 90.

7. Catheter according to one or more of the preceding claims, characterized by that the membrane (14) comprises synthetic resin alloys such as polytetrafluoroethylene.

8. Catheter according to one or more of the preceding claims, characterized by that the membrane guide (16) in the form of a ring (40) includes an inner bearing surface (48), an outwardly extending flange (42), and a connecting ridge (44).

9. Catheter according to claim 8, characterized by that the outer diameter of connecting ridge (44) is sufficiently larger than the inner diameter of membrane (14) such that a "force-fit" engagement between membrane (14) and guide (16) is maintained.

10. Catheter according to claims 8 or 9, characterized by that a snap ring is utilized to ensure secure attachment of membrane (14) to membrane guide (16).

11. Catheter according to one or more of the preceding claims, characterized by that the membrane guide (16) comprises a material having a relatively low frictional coefficient, such as polyethylene or polytetrafluoroethylene based materials.

12. Catheter according to one or more of the preceding claims, characterized by that the inner bearing surface (48) of the membrane guide (16) exhibits sufficient lubricity to easily pass along the outside of catheter tube (12) during use.

13. Catheter according to one or more of the preceding claims, characterized by the diameter of catheter tube (12) at the leading edge (20) being in the range of about 5 mm, and the diameter of membrane (14) and the inner diameter of membrane guide (16) being at least about 6 mm.

14. Catheter according to one or more of the preceding claims, characterized by that the catheter (10) is utilized as a probe for medical techniques.

15. Catheter according to one or more of the preceding claims, charaterized by that the trailing edge (22) of the catheter tube (12) includes a coupler (26) securely affixed thereto, coupler (26) being configured to facilitate adaption of the catheter tube (12) to the opening of a conventional urine collection bag.

## Patentansprüche

1. Katheter zum Einführen in einen anatomischen Kanal, welcher Katheter umfaßt:
- einen allgemein hohlen Katheterschlauch (12) mit einer voreilenden und einer nacheilenden Kante (20, 22), die jeweils gegenüberliegend angeordnet sind, einen axialen Hohlraum (21), der sich über dessen Länge erstreckt,
- wobei der Katheterschlauch (12) eine Spitze (20a) hat, die die voreilende Kante (20) des Katheterschlauches (12) umfaßt und ausgebildet ist, eine weiche Struktur zu haben,
- wobei der Katheterschlauch (12) aus einem flexiblen Polymer-Material hergestellt ist, das eine ausreichende Stärke hat, daß der Katheterschlauch (12) während eines Einführens nicht zusammengedrückt wird, und auch eine ausreichende Elastizität hat, um es zu gestatten, daß der Katheter (10) während des Einführens in den anatomischen Kanal lenkbar ist,
- eine längliche, rohrförmige und ausreichend elastische Membran (14), die innerhalb des Hohlraums (21) angeordnet ist und ein erstes und ein zweites offenes Ende (34, 36) und einen sich dazwischen ausdehnenden Körper (38) hat,
- wobei die Membran (14) aus einem dünnen Polymer-Material hergestellt ist, das eine ausreichende Gleitfähigkeit hat, um sanft aus der Öffnung von Spitze (21a) heraus und über die voreilende Kante (20) des Katheterschlauches (12) zu gleiten und nachfolgend relativ zu dem Äußeren des Katheterschlauchs (12) zurückzugleiten,
- eine Membranführung (16), die in der Form eines Rings (40) ausgebildet ist und für eine axiale Bewegung über das Äußere des Katheterschlauchs (12) geeignet ausgebildet ist, die außerdem aus einem Kunststoffmaterial mit einem relativ niedrigen Reibungskoeffizienten hergestellt ist, wobei das erste Ende (34) der Membran (14) sicher an der Membranführung (16) befestigt ist,
- wobei die jeweiligen Größen der Membranführung (16) und der Membran (14) so gewählt sind, daß deren bequeme Bewegung über die Außenfläche des Katheterschlauches (12) vereinfacht wird,
- wobei die Membranführung (16) und das erste Ende (34) der Membran um das Äußere der voreilenden Kante (20) des Schlauches angeordnet sind und das zweite Ende (36) der Membran und ein Hauptanteil des Membrankörpers (38) innerhalb des Schlauchhohlraums (21) positioniert sind, so daß, wenn der Katheter (10) manuell in den anatomischen Kanal eingeführt wird, ein Drücken des Katheterschlauches (12) in den Kanal zu einem kontinuierlichen Herausziehen der Membran (14) aus dem Katheterschlauch (12) führt, so daß zuletzt die Membran (14) aus dem Schlauchhohlraum (21) herausgezogen und zwischen dem Schlauch (12) und dem anatomischen Kanal angeordnet ist.

2. Katheter nach Anspruch 1, dadurch gekennzeichnet, daß der Außendurchmesser des Katheterschlauches (12) im Bereich von etwa 5,08 bis 7,6 mm, insbesondere etwa 6,35 mm, liegt, wenn der Katheter (10) als Harnkatheter verwendet wird.

3. Katheter nach Anspruch 1 oder 2, dadurch gekennzeichnet, daß der Durchmesser des Hohlraums (21) des Katheterschlauches (12) im Bereich von etwa 2,54 mm bis etwa 5,08 mm liegt.

4. Katheter nach einem oder mehreren der vorhergehenden Ansprüche, dadurch gekennzeichnet, daß der Radius der Spitze (20) des Katheterschlauches (12) für einen Katheterschlauch (12) mit einem Durchmesser von etwa 6,35 mm in dem Bereich von 1,27 mm liegt.

5. Katheter nach einem oder mehreren der vorhergehenden Ansprüche, dadurch gekennzeichnet, daß der Katheterschlauch (12) aus Polyvinylchlorid (PVC), insbesondere Polyvinylchlorid medizinischer Qualität, hergestellt ist.

6. Katheter nach einem oder mehreren der vorhergehenden Ansprüche, dadurch gekennzeichnet, daß der Katheterschlauch (12) ein Polymer-Material aufweist, das eine Shore-A-Härte im Bereich von etwa 70 bis 160 und bevorzugt von etwa 90 hat.

7. Katheter nach einem oder mehreren der vorhergehenden Ansprüche, dadurch gekennzeichnet, daß die Membran (14) synthetische Harz-Verbindungen wie Polytetrafluoroethylen aufweist.

8. Katheter nach einem oder mehreren der vorhergehenden Ansprüche, dadurch gekennzeichnet, daß die Membranführung (16) in der Form eines Rings (40) eine innere Lagerfläche (48), einen sich nach außen erstreckenden Flansch (42) und eine Verbindungsrippe (44) aufweist.

9. Katheter nach Anspruch 8, dadurch gekennzeichnet, daß der Außendurchmesser der Verbindungsrippe (44) ausreichend größer als der Innendurchmesser der Membran (14) ist, so daß ein "kraftschlüssiger" Eingriff zwischen der Membran (14) und der Führung (16) aufrechterhalten wird.

10. Katheter nach Anspruch 8 oder 9, dadurch gekennzeichnet, daß ein Schnappring verwendet wird, um eine sichere Anbringung der Membran (14) an der Membranführung (16) zu gewährleisten.

11. Katheter nach einem oder mehreren der vorhergehenden Ansprüche, dadurch gekennzeichnet, daß die Membranführung (16) ein Material mit einem relativ niedrigen Reibungskoeffizienten aufweist, wie Materialien, die auf Polyethylen oder Polytetrafluoroethylen basieren.

12. Katheter nach einem oder mehreren der vorhergehenden Ansprüche, dadurch gekennzeichnet, daß die innere Lagerfläche (48) der Membranführung (16) ausreichende Gleitfähigkeit zeigt, um sich während eines Gebrauchs einfach entlang der Außenseite des Katheterschlauches (12) zu bewegen.

13. Katheter nach einem oder mehreren der vorhergehenden Ansprüche, dadurch gekennzeichnet, daß der Durchmesser des Katheterschlauches (12) an der voreilenden Kante (20) in dem Bereich von etwa 5 mm liegt und der Durchmesser der Membran (14) und der Innendurchmesser der Membranführung (16) mindestens etwa 6 mm beträgt.

14. Katheter nach einem oder mehreren der vorhergehenden Ansprüche, dadurch gekennzeichnet, daß der Katheter (10) als eine Meßeinrichtung für medizinische Techniken verwendet wird.

15. Katheter nach einem oder mehreren der vorhergehenden Ansprüche, dadurch gekennzeichnet, daß die nacheilende Kante (22) des Katheterschlauches (12) eine daran sicher befestigte Kuppeleinrichtung (26) aufweist, wobei die Kuppeleinrichtung (26) zur vereinfachung des Anschlusses des Katheterschlauches (12) an die Öffnung eines herkömmlichen Harnsammelbehälters ausgebildet ist.

## Revendications

1. Cathéter pour insertion dans un conduit anatomique comprenant :
- un tube de cathéter (12) généralement creux ayant des arêtes avant et arrière (20, 22) disposées respectivement opposées, une lumière axiale (21) s'etendant à travers la longueur de celui-ci,
- le tube de cathéter (12) ayant une pointe (20a) qui comprend l'arête avant (20) du tube de cathéter (12) et qui est configurée pour avoir une configuration douce,
- le tube de cathéter (12) étant fait en un matériau polymère flexible ayant une résistance suffisante pour que le tube de cathéter (12) ne s'affaisse pas pendant l'insertion ainsi qu'une élasticité suffisante pour permettre de manoeuvrer le cathéter pendant l'insertion dans le conduit anatomique,
- une membrane élastique (14) tubulaire allongée et suffisamment élastique disposée à l'intérieur de la lumière (21) ayant des première et seconde extrémités ouvertes (34, 36) et un corps (38) traversant entre les deux ;
- ladite membrane (14) étant faite d'un matériau polymère mince ayant un pouvoir lubrifiant suffisant pour glisser doucement à l'extérieur de l'ouverture de la pointe (21a) et par dessus l'arête arrière (20) du tube de cathéter (12) et pour ensuite reglisser par rapport à l'extérieur du tube de cathéter (12),
- un guide de membrane (16) étant configuré en forme d'anneau (40) et configuré de manière appropriée pour un mouvement axial près de l'extérieur du tube de cathéter (12), étant de plus fait en un matériau plastique ayant un coefficient de friction relativement bas, la première extrémité (34) de la membrane (14) étant fixée de manière sûre audit guide de membrane (16),
- les grandeurs respectives du guide de membrane (16) et de la membrane (14) étant choisies de telle manière que leur mouvement approprié sur la surface extérieure du tube de cathéter (12) est facilité,
- dans lequel ledit guide de membrane (16) et ladite première extrémité de membrane (34) sont disposés près de l'extérieur de ladite arête avant (20) du tube, et ladite seconde extrémité de membrane (36) et une plus grande portion dudit corps de membrane (38) sont positionnées à l'intérieur de ladite lumière (21) du tube, de telle manière que, lorsque le cathéter (10) est inséré manuellement dans le conduit anatomique, le fait de pousser le tube de cathéter (12) dans ledit conduit aura pour résultat une extraction continue de la membrane (14) du tube (12) du cathéter si bien que finalement ladite membrane (14) sera retirée de ladite lumière (21) du tube et sera interposée entre ledit tube (12) et ledit conduit anatomique.

2. Cathéter selon la revendication 1, caractérisé en ce que le diamètre extérieur du tube de cathéter (12), lorsque le cathéter (10) est utilisé comme cathéter urinaire, est de l'ordre d'environ 5,08 à 7,6 mm, en particulier est d'environ 6,35 mm.

3. Cathéter selon la revendication 1 ou 2, caractérisé en ce que le diamètre de la lumière (21) du tube du cathéter (12) est de l'ordre d'environ 2,54 mm à environ 5,08 mm.

4. Cathéter selon l'une ou plusieurs des revendications précédentes, caractérisé en ce que le rayon de la pointe (20) du tube de cathéter (12) est de l'ordre de 1,27 mm pour un tube de cathéter (12) ayant un diamètre d'environ 6,35 mm.

5. Cathéter selon l'une ou plusieurs des revendications précécentes, caractérisé en ce que le tube de cathéter (12) est fait en chlorure de polyvinyle (PVC), en particulier en chlorure de polyvinyle à usage médical.

6. Cathéter selon l'une ou plusieurs des revendications précédentes, caractérisé en ce que le tube de cathéter (12) comprend une matière polymère ayant un degré de dureté Shore A de l'ordre d'environ 70 à 160, et de préférence d'environ 90.

7. Cathéter selon l'une ou plusieurs des revendications précédentes, caractérisé en ce que la membrane (14) comprend des alliages de résine synthétique comme le polytétrafluoréthylène.

8. Cathéter selon l'une ou plusieurs des revendications précédentes, caractérisé en ce que le guide de membrane (16) en forme d'anneau (40) comprend une surface d'appui intérieure (48), une collerette qui s'etend vers l'extérieur (42) et une crête de connexion (44).

9. Cathéter selon la revendication 8, caractérisé en ce que le diamètre extérieur de la crête de connexion (44) est suffisamment plus grande que le diamètre intérieur de la membrane (14) de telle manière qu'un engrènement par ajustement serré est maintenu entre la membrane (14) et le guide (16).

10. Cathéter selon la revendication 8 ou 9, caractérisé en ce qu'un circlip extérieur est utilisé pour assurer la fixation sûre de la membrane (14) et du guide de membrane (16).

11. Cathéter selon l'une ou plusieurs des revendications précédentes, caractérisé en ce que le guide de membrane (16) comprend une matière ayant un coefficient de friction relativement bas tel que des matières à base de polyéthylène ou de polytétrafluoréthylène.

12. Cathéter selon l'une ou plusieurs des revendications précédentes, caractérisé en ce que la surface d'appui intérieure (48) du guide de membrane (16) montre un pouvoir lubrifiant suffisant pour passer le long de l'extérieur du tube de cathéter (12) pendant l'utilisation.

13. Cathéter selon l'une ou plusieurs des revendications précédentes, caractérisé par le fait que le diamètre du tube de cathéter (12) sur l'arête avant (20) est de l'ordre d'environ 5 mm et le diamètre de la membrane (14) et le diamètre du guide de membrane (16) est d'au moins environ 6 mm.

14. Cathéter selon l'une ou plusieurs des revendications précédentes, caractérisé en ce que le cathéter (10) est utilisé comme sonde pour techniques médicales.

15. Cathéter selon l'une ou plusieurs des revendications précécentes, caractérisé en ce que l'arête arrière (22) du tube de cathéter (12) comprend un coupleur (26) fixé de manière sûre à celle-ci, le coupleur (26) étant configuré pour faciliter l'adaptation du tube de cathéter (12) à l'ouverture d'une poche conventionnelle de recueil d'urine.
